# EUROPÄISCHE PATENTSCHRIFT

(11) **EP 2 893 899 B1**
(45) Veröffentlichungstag und Bekanntmachung des Hinweises auf die Patenterteilung: **16.11.2016**
(21) Anmeldenummer: 15150482.6
(22) Anmeldetag: 08.01.2015
(51) Int. Cl.: A61B 90/00, A61B 90/70, A61B 90/90, A61B 90/98

(54) **Spül-, Sterilisations- oder Waschautomat, insbesondere Reinigungs- und Desinfektionsgerät**
Rinsing, sterilization or washing machine, in particular a cleaning and disinfecting device
Automate de rinçage, de lavage ou de stérilisation, en particulier appareil de nettoyage et de désinfection

(30) Priorität: 13.01.2014 DE 102014100297
(43) Veröffentlichungstag der Anmeldung: 15.07.2015
(73) Patentinhaber: Miele & Cie. KG, 33332 Gütersloh (DE)
(72) Erfinder: Schröder, Frank, 32139 Spenge (DE); Rummler, Britta, 33604 Bielefeld (DE); Kukolja, Janko, 33615 Bielefeld (DE); Bambeck, Daniel, 45149 Essen (DE); Berling, Sven, 42553 Velbert (DE); Blömer, Josef, 45473 Mülheim an der Ruhr (DE); Mracek, Maik, 33334 Gütersloh (DE); Bertling, Andre, 33442 Herzebrock-Clarholz (DE)

(56) Entgegenhaltungen:
- DE-A1- 10 138 850
- DE-A1- 19 917 206
- DE-A1-102012 211 282
- JP-A- 2004 283 339
- JP-A- 2009 010 865
- US-A1- 2009 220 377
- US-A1- 2012 212 330

## Beschreibung

Die Erfindung betrifft einen Spül-, Sterilisations- oder Waschautomaten mit den Merkmalen des Oberbegriffs von Anspruch 1.

Spül-, Sterilisations- oder Waschautomaten im Allgemeinen sowie Reinigungs- und Desinfektionsgeräte für medizinische Instrumente im Speziellen sind aus dem Stand der Technik an sich bekannt, weshalb es eines gesonderten druckschriftlichen Nachweises an dieser Stelle nicht bedarf.

Gattungsgemäße Spülautomaten verfügen über einen Spülbehälter. Dieser stellt einen Behandlungsraum, d.h. einen Spülraum bereit, der im bestimmungsgemäßen Verwendungsfall das zu reinigende Spülgut aufnimmt. Innerhalb des Spülbehälters ist eine Sprüheinrichtung ausgebildet, die vorzugsweise über verdrehbar angeordnete Sprüharme verfügt, die der Beschickung des zu reinigenden Spülguts mit Spülflotte dienen.

Zur Beladung des Spülraums mit zu reinigendem Spülgut ist der Spülbehälter mit einer Beschickungsöffnung ausgerüstet. Diese ist verwenderseitig mittels einer Tür fluiddicht verschließbar, wobei die Tür bewegbar ausgebildet ist und entweder horizontal oder vertikal verfahrbar oder um eine Schwenkachse verdrehbar ist.

Zu den gesetzlichen und normativen Anforderungen bei der Aufbereitung von insbesondere medizinischem Instrumentarium und/oder Laborutensilien gehört unter anderem die lückenlose Dokumentation der Prozess- und Chargendaten des Spülgutkreislaufes inklusive einer Benutzererkennung. Die Aufbereitung, das heißt Reinigung, Desinfektion und Sterilisation von Medizinprodukten muss mittels validierter Verfahren erfolgen und entsprechend dokumentiert werden.

Aus dem Stand der Technik ist es in diesem Zusammenhang bekannt geworden, die zum Zwecke der Spülgutreinigung typischerweise zum Einsatz kommenden Spülkörbe und/oder-wagen mittels einer Magnetleiste auszurüsten. Eine solche Magnetleistenkodierung gestattet eine automatische Spülwagenerkennung und anschließende Zuordnung eines entsprechenden Spülprogramms. Es können Fehlbedienungen insoweit wirkungsvoll vermieden werden.

Von Nachteil bei der vorbekannten Magnetleistenkodierung ist allerdings, dass die Dokumentation der Daten weitgehend unabhängig von der tatsächlichen Spülwagen-Beladung erfolgt, denn die Dokumentation erfolgt chargenbezogen und gibt keine direkten Informationen über den eigentlichen Inhalt einer Charge. Um hier Abhilfe zu schaffen, erfolgt in Ergänzung zur Spülwagenkodierung eine Barcodekodierung einzelner Chargeneinheiten, wobei derartige Barcodes im Verlauf eines Aufbereitungsprozesses manuell einzuscannen sind. Die tatsächliche Beladung kann aber auch bei einer zusätzlichen Barcodekodierung nicht immer vollständig und richtig erfasst werden, weshalb das Bedienpersonal stets darauf zu achten hat, dass die Beladung mit der aktuellen Kodierung tatsächlich übereinstimmt. Insoweit ist das vorbekannte System sehr fehleranfällig und auch Lücken und/oder Widersprüche in der Dokumentation können nicht vollends vermieden werden.

Hinzu kommt, dass das bestehende System der Spülwagenkodierung mittels Magnetleiste in der Anzahl der Programmiermöglichkeiten sehr begrenzt ist.

Aus der DE 19917206 A1 ist ein Reinigungs- und Desinfektionsautomat mit einer Vorrichtung zur Identifikation von medizinischen Instrumenten bekannt. Dabei sind die in den Automat geladenen medizinischen Instrumente jeweils mit Datenträgern bestückt, welche von einer Sende/Empfangseinrichtung bei geschlossener Gerätetür ausgelesen werden.

Es ist ausgehend vom Vorbeschriebenen Aufgabe der Erfindung, einen Spül-, Sterilisations- oder Waschautomaten der eingangs genannten Art dahingehend weiterzuentwickeln, dass eine fehlerfreie und lückenlose Detektion von im Behandlungsraum befindlichem Behandlungsgut gestattet ist.

Zur Lösung dieser Aufgabe wird mit der Erfindung ein Spül-, Sterilisations- oder Waschautomat mit den Merkmalen von Anspruch 1 vorgeschlagen.

Mit der erfindungsgemäßen Ausgestaltung wird ein im Unterschied zum vorbekannten Magnet-System völlig anders aufgebautes Detektionssystem vorgeschlagen. Das Detektionssystem nach der Erfindung verfügt einerseits über einen Identifikator sowie andererseits über eine Leseeinheit, die dazu ausgerüstet ist, den Identifikator identifizieren zu können. Dabei arbeitet das System völlig berührungslos, vorzugsweise auf RFID-Basis.

Der Vorteil eines funkwellenbasierten Detektionssystems liegt darin, dass eine beliebige Anzahl von entsprechenden Identifikatoren eingesetzt werden kann, was es gestattet, einzelne Chargen sowie deren Inhalte genau identifizieren zu können. So erlaubt das erfindungsgemäße System nicht nur die Identifikation einzelner Spül-, Sterilisations- oder Waschmodule, z.B. Spülwagen und/oder-körbe, es können auch einzelne Aufbewahrungseinrichtungen innerhalb solcher Spül-, Sterilisations- oder Waschmodule, insbesondere innerhalb solcher Spülwagen und/oder-körbe, wie zum Beispiel Siebschalen und/oder dergleichen identifiziert werden. Je nach Größe der eingesetzten Identifikatoren lassen sich sogar einzelne aufzubereitende medizinische Instrumente eineindeutig zuordnen. Im Ergebnis ist so eine lückenlose und fehlerfreie Dokumentation des gesamten Aufbereitungsprozesses möglich.

Die RFID-Technologie ist aus dem Stand der Technik an sich bekannt. Sie findet beispielsweise bei Durchlaufgeschirrspülmaschinen zur Spülgutidentifikation Verwendung, wie dies beispielsweise mit der DE 10 2008 033 741 A1 beschrieben ist. Die bei Durchlaufgeschirrspülmaschinen insoweit zum Einsatz kommende Konstruktion kann aber nicht auf Spül-, Sterilisations- oder Waschautomaten der gattungsgemäßen Art, das heißt auf Nicht-Durchlaufgeschirrspülmaschinen übertragen werden. Dies deshalb nicht, weil der Spül-, Sterilisations- oder Waschbehälter eines gattungsgemäßen Spül-, Sterilisations- oder Waschautomaten im Verwendungszustand einer ordnungsgemäßen Benutzung einen sogenannten faradäischen Käfig ausbildet, womit der Einsatz von Funktechnologie dem Grunde nach ausgeschlossen ist. Bei Durchlaufgeschirrspülmaschinen ist dies anders, da hier eine Detektionserfassung eingangsseitig der Durchlaufgeschirrspülmaschine stattfinden kann, das heißt im Moment der Spülguteinfuhr.

Um gleichwohl gattungsgemäße Spül-, Sterilisations- oder Waschautomaten mit einem funkbasierenden Detektionssystem auszurüsten, wird mit der Erfindung konstruktiv vorgeschlagen, dass die Leseeinheit relativ bewegbar zum Behandlungsraum angeordnet ist.

Durch diese konstruktive Maßnahme wird es möglich, das vom Behandlungsraum des Spül-, Sterilisations- oder Waschautomaten aufgenommene Behandlungsgut zu "scannen". So gestattet es die bewegbare Anordnung der Leseeinheit, dass diese an dem mit einem oder mehreren Identifikatoren ausgerüsteten Behandlungsgut vorbeibewegt wird, infolge dessen es zu einer vollständigen Erfassung sämtlicher am Behandlungsgut angeordneter Identifikatoren kommt. Es wird so eine 100 %ige Erfassung sichergestellt, so dass auch die hieraus abgeleitete Dokumentation fehlerfrei und lückenlos ist.

Die Leseeinheit ist gemäß einem weiteren Merkmal der Erfindung zwecks bewegbarer Anordnung relativ zum Behandlungsgut in die vom Spül-, Sterilisations- oder Waschautomaten bereitgestellte Tür integriert. Es wird so in konstruktiv eleganter und einfacher Weise die relative Bewegbarkeit der Leseeinheit zum vom Behandlungsraum aufgenommenen Behandlungsgut realisiert. Sobald nach einer ordnungsgemäßen Beschickung des Behandlungsraums zum fluiddichten Verschluss des Behandlungsraums die Tür des Behandlungsraums verwenderseitig geschlossen wird, erfolgt eine Vorbeibewegung der von der Tür des Behandlungsraums aufgenommenen Leseeinheit am Behandlungsgut vorbei, was in der schon vorbeschriebenen Weise zu einem Abtasten und Einscannen der im Behandlungsraum befindlichen Identifikatoren führt. Dabei kann die Tür sowohl verfahr- oder verschwenkbeweglich ausgebildet sein, und zwar hinsichtlich der verfahrbeweglichen Ausgestaltung sowohl in vertikaler als auch in horizontaler Richtung, wobei insbesondere die Anordnung der Leseeinheit an die Verfahrrichtung der Tür derart angepasst ist, dass die Leseeinheit beim Schließen der Tür über annähernd die gesamte Erstreckung der Beschickungsöffnung in Verfahrrichtung vorbeibewegt werden kann.

Spül-, Sterilisations- oder Waschautomaten der gattungsgemäßen Art verfügen typischerweise über eine Tür mit einem Sichtfenster. Die Tür ist zu diesem Zweck mit einem Durchbruch versehen, in den eine Glasscheibe zum fluiddichten Verschluss des Durchbruches eingesetzt ist. Diese Glasscheibe verfügt bevorzugterweise über zwei unter Belassung eines Spaltraums beabstandet voneinander angeordnete Einzelscheiben. Für Anwendungen, bei denen ein Sichtfenster nicht erforderlich oder gewünscht ist, kann zum fluiddichten Verschluss des Durchbruches der Tür statt der Glasscheibe eine alternative Abdeckung vorhanden sein, welche über zwei unter Belassung eines Spaltraums beabstandet voneinander angeordnete Abdeckungselemente verfügt. Dabei ist das dem Behandlungsraum zugewandte Abdeckungselement zumindest bereichsweise nichtmetallisch ausgebildet, insbesondere aus Glas oder Kunststoff. Mit der Erfindung wird vorgeschlagen, die Leseeinheit in diesem Spaltraum, das heißt zwischen den Abdeckungselementen der Abdeckung, bzw. zwischen den Einzelscheiben der Glasscheibe anzuordnen. Diese Ausgestaltung ist in zweierlei Hinsicht von Vorteil. Zum einen ist die Detektionsseite der Leseeinheit nur durch eines der Abdeckungselemente, insbesondere den nichtmetallischen Bereich des dem Behandlungsraum zugewandten Abdeckungselements, bzw. eine Einzelscheibe vom abzutastenden Behandlungsraum getrennt, so dass auf die von der Leseeinheit abgegebenen Funksignale kein oder nahezu kein materialbedingt störender Einfluss ausgeübt ist, womit ein einwandfreies Arbeiten des Detektionssystems sichergestellt ist. Zum anderen ist insbesondere bei einer verfahrbar ausgebildeten Tür eine geometrisch optimierte Ausrichtung der Leseeinheit sichergestellt, da diese frontseitig des Behandlungsraums bei einer Verfahrbewegung der Tür entlang bewegt wird, das heißt die gesamte Beschickungsöffnung mittels der Leseeinheit vollständig überfahren wird. Übertragungsverluste können so ebenso wie Fehlerfassungen auf ein Minimum reduziert werden, so dass auch insoweit eine fehlerfreie und lückenlose Identifikatorenerfassung sichergestellt ist.

Die Leseeinheit weist gemäß einem weiteren Merkmal der Erfindung mehrere Antennen auf. Es sind bevorzugterweise drei Antennen vorgesehen. Bei diesen Antennen handelt es sich vorzugsweise um Planarantennen. Dabei ist der Einsatz von Planarantennen insbesondere deshalb bevorzugt, weil sie einerseits im Spaltraum zwischen den Einzelscheiben der Glasscheibe der Tür Platz finden und weil sie andererseits ein zur vollständigen Detektion des Spülraums optimiert ausgebildetes Strahlungsfeld bereitstellen. Dabei ist der Einsatz von mehreren Antennen, insbesondere drei Antennen deshalb bevorzugt, damit sich in Überlagerung der einzelnen Strahlungsbereiche eine vollständige Erfassung des Behandlungsrauminnenraums ergibt und so auch einzelne Chargeninhalte oder sogar einzelne Instrumente sicher erfasst werden können, die in Beschickungsrichtung im Behandlungsraum hintereinander angeordnet sind.

Die Leseeinheit ist gemäß einem weiteren Merkmal der Erfindung von einem Gehäuse aufgenommen. Bei diesem Gehäuse kann es sich bevorzugterweise um einen Rahmen handeln, der die Leseeinheit beziehungsweise deren einzelne Baukomponenten aufnimmt. Dabei dient ein solches Gehäuse insbesondere zum Zwecke der ordnungsgemäßen Positionierung und der damit einhergehenden ordnungsgemäßen Ausrichtung der von der Leseeinheit bereitgestellten Antennen. Insbesondere aus Gründen der vereinfachten Montage ist der Einsatz eines solchen Gehäuses von Vorteil.

Das Gehäuse ist gemäß einem weiteren Merkmal der Erfindung auf seiner dem Behandlungsraum abgewandten Rückseite abgeschirmt ausgebildet. Es wird dadurch erreicht, dass es nicht zu einer ungewollten Erfassung von außerhalb des Behandlungsraums befindlichen Identifikatoren kommt. Eine Fehlfassung wird so vermieden.

Die rückseitig des Gehäuses vorgesehene Abschirmung verfügt gemäß einem weiteren Merkmal der Erfindung über einen Durchbruch, womit es gestattet ist, seitens der Leseeinheit eine benutzerseitige Erkennung zu detektieren. Das erfindungsgemäße Detektionssystem kann mithin nicht nur in vorbeschriebener Weise eine Behandlungsgutidentifikation vornehmen, sondern auch einen benutzerseitig zu tragenden Identifikator erfassen. Dabei erfolgt diese Erfassung durch die Ausnehmung in der gehäuserückseitig vorgesehenen Abschirmung.

Mit der Erfindung wird insgesamt eine Konstruktion vorgeschlagen, wonach die Leseeinheit in die Glasscheibe des Spül-, Sterilisations- oder Waschautomatenintegriert ist. Durch die Integration der Leseeinheit in die Tür kann in vorteilhafter Weise auf den Einbau der Leseeinheit in den Behandlungsraum und die damit verbundenen Modifikationen des Behandlungsraums verzichtet werden. Die Leseeinheit kann im Übrigen innerhalb der Glasscheibe wartungsfrei betrieben werden und dies ohne jeden störenden Einfluss auf einen Aufbereitungsprozess. Dies gestattet es auch, die RFID-Überwachung als optionales Nachrüstprodukt anzubieten. Die Leseeinheit ist bevorzugterweise in den Zwischenraum der Thermopenscheiben integriert und das Interface zum CAN-BUS ist vom Geräteschaltschrank des Spül-, Sterilisations- oder Waschautomatenaufgenommen. Der Beladevorgang des Behandlungsraums bleibt insgesamt unverändert und die Erfassung der Identifikatoren, das heißt der sogenannten TAGs erfolgt beim Schließen der Tür, ohne einen weiteren Arbeitsschritt durch den Benutzer.

Als Antennen werden drei Planarantennen eingesetzt, die in direkter Nähe zur Glasscheibe betrieben werden, wobei die elektrische Beeinflussung durch die Glasscheibe in die Parameter der Antennen miteingerechnet ist. Die Antennen sind bevorzugterweise im unteren Teil der Glasscheibe derart angebracht, dass die Antennen beim Schließen der Tür zum einen am Spülkorb sowie zum anderen an etwaigen Siebschalen und/oder Instrumenten entlang fahren. Die Hauptstrahlungsrichtung der Antennen führt somit direkt in den Behandlungsraum hinein. Eine unerwünschte Erfassung von anderem, nicht im Behandlungsraum befindlichen Behandlungsgut wird damit weitgehend ausgeschlossen. Es kann jedoch unter Umständen durch unerwünschte Reflexionen zu sporadischen Fehlerfassungen außerhalb des Behandlungsraums kommen. Um dies zu verhindern, sind die Antennen in ein Gehäuse eingesetzt, welches die gesamte Breite des Behandlungsraums abdeckt. Das Gehäuse ist vorzugsweise als Rahmen ausgebildet, das zur Verringerung der Funkabstrahlung auf der Rückseite abgeschirmt ist. Die Antennen sind insbesondere derart im unteren oder einem seitlichen Bereich der Tür angeordnet, dass sie beim Bewegen der Tür von der vollständig geöffneten Stellung in die geschlossene Stellung am Behandlungsraum nahezu über dessen gesamte Breite oder dessen gesamte Höhe entlangfahren können. Senkrecht zu dieser Bewegungsrichtung sind mehrere, insbesondere drei Antennen neben- oder übereinander angeordnet, so dass das Scannen insgesamt flächig über annähernd die gesamte Fläche der Beschickungsöffnung erfolgen kann.

Die Anordnung der Antennen innerhalb der Tür hat zudem den Vorteil, dass sie auch im Falle eines unachtsamen Beladens nicht beschädigt werden. Insgesamt ist das Detektionssystem wartungsfrei.

Bestimmte Typen der erfindungsgemäßen Spül-, Sterilisations- oder Waschautomaten verfügen nicht über lediglich eine einzige Tür, sondern über zwei, in der Regel an gegenüberliegenden Seiten des Behandlungsraums angeordnete Türen. Eine dieser Türen ist dabei der sogenannten "unreinen" Seite zugewandt, von wo aus eine Bestückung des Spül-, Sterilisations- oder Waschbehälters mit dem zu behandelnden Behandlungsgut stattfindet, während die andere Tür der sogenannten "reinen" Seite zugewandt ist, von wo aus das Behandlungsgut nach Ablauf des Spül-, Sterilisations- oder Waschprogramms wieder aus dem Spül-, Sterilisations- oder Waschbehälter entnommen wird. Bei einer vorteilhaften Ausführungsform ist in beide Türen jeweils eine Leseeinheit integriert. Beide Leseeinheiten können insbesondere jeweils wie zuvor beschrieben ausgebildet und angeordnet sein. Durch das Vorhandensein zweier Leseeinheiten wird die Erfassung der Identifikatoren noch sicherer, eine Fehl- oder Nichterfassung von Identifikatoren kann praktisch ausgeschlossen werden.

Weitere Merkmale und Vorteile der Erfindung ergeben sich aus der nachfolgenden Beschreibung anhand der Figuren. Dabei zeigen
- Fig. 1: in schematischer Seitenansicht einen Spülautomaten nach der Erfindung;
- Fig. 2: in schematischer Frontansicht die Tür des Spülautomaten nach Fig. 1 und
- Fig. 3: in einem schematischen Blockdiagramm das erfindungsgemäße Detektionssystem.

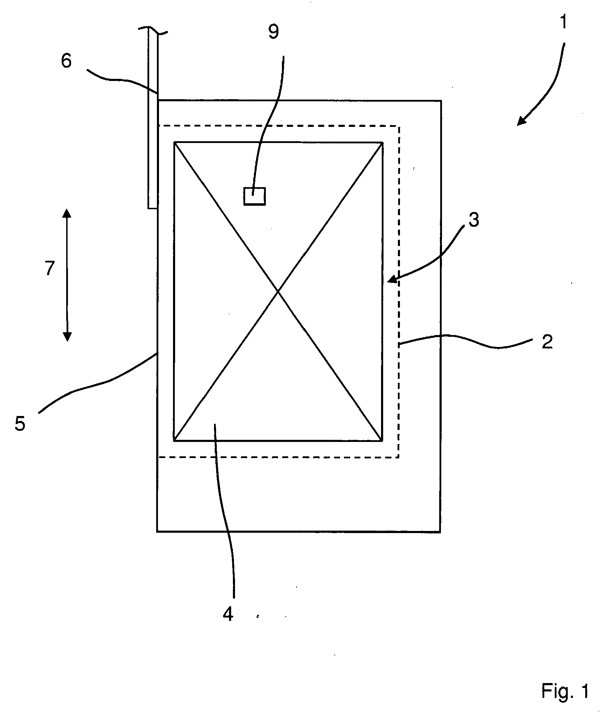

Fig. 1 lässt in schematischer Ansicht einen Spül-, Sterilisations- oder Waschautomaten 1 ohne Beschränkung der Allgemeinheit in der Ausgestaltung als Reinigungs- und Desinfektionsgerät für medizinische Instrumente erkennen, im Folgenden als Spülautomat 1 bezeichnet. Der Spülautomat 1 verfügt über einen Spülbehälter 2, der seinerseits einen Behandlungsraum, nämlich Spülraum 3 bereitstellt. Über eine Beschickungsöffnung 5 ist der Spülraum 3 zugänglich, wobei für einen fluiddichten Verschluss der Beschickungsöffnung 5 eine Tür 6 vorgesehen ist. Im gezeigten Ausführungsbeispiel ist die Tür 6 in Höhenrichtung 7 verfahrbar ausgebildet.

Im bestimmungsgemäßen Verwendungsfall nimmt der Spülraum 3 des Spülautomaten 1 zu reinigendes Behandlungsgut, nämlich Spülgut 4 auf. Bei diesem Spülgut 4 kann es sich beispielsweise um medizinische Instrumentarien und/oder Laborutensilien handeln, die zur ordnungsgemäßen Ausrichtung und Positionierung innerhalb des Spülraums 3 von einem Spülgutwagen beziehungsweise von Spülkörben und/oder -einsatzsieben aufgenommen sind. Zur Erkennung einzelner Spülwagen und/oder -körbe und/oder -siebe und/oder einzelner Spülgüter ist ein Detektionssystem 8 vorgesehen, das unter anderem Identifikatoren 9 in Form von zum Beispiel Transpondern umfasst. Die Darstellung nach Fig. 1 lässt exemplarisch einen Identifikator 9 erkennen.

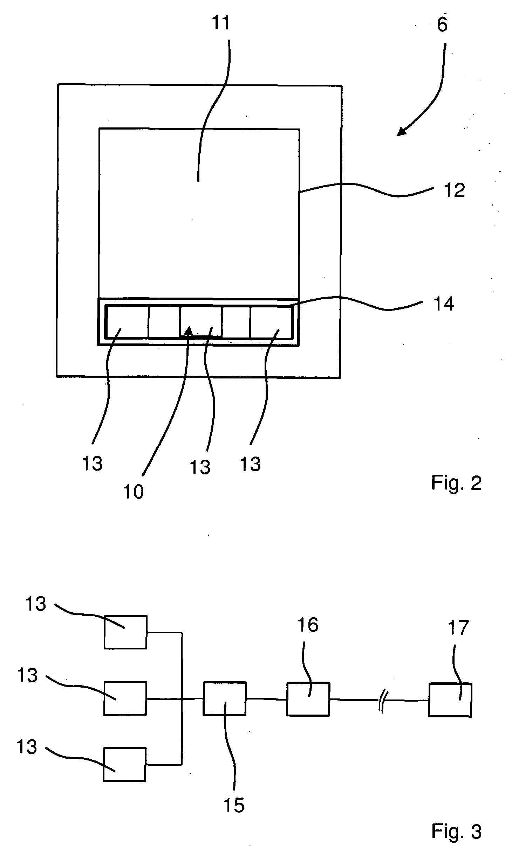

Das erfindungsgemäße Detektionssystem 8 verfügt des Weiteren über eine Leseeinheit 10, welche ihrerseits im gezeigten Ausführungsbeispiel drei Planarantennen 13 umfasst, über einen Antennenumschalter 15 sowie eine Leseelektronik 16, wie sich dies insbesondere aus der Schemazeichnung nach Fig. 3 ergibt. Die Leseeinheit 10 ist von der Tür 6 des Spülautomaten 1 getragen. Es ist dabei vorgesehen, dass die Leseeinheit im Spaltraum zwischen den beiden beabstandet voneinander angeordneten Einzelscheiben der Glasscheibe 11 der Tür 6 angeordnet ist. Zur positionssicheren Anordnung der Leseeinheit kommt ein Gehäuse 14 in Form eines Rahmens zum Einsatz, wie dies insbesondere die Darstellung nach Fig. 2 erkennen lässt.

Zur Auswertung der von der Leseeinheit 10 gelieferten Signale ist ferner eine Anschaltelektronik 17 vorgesehen, die im nicht näher gezeigten Geräteschaltschrank des Spülautomaten 1 untergebracht ist. Der Antennenumschalter 15 und die Leseelektronik 16 können zusammen mit der Leseeinheit 10 im Gehäuse 14 untergebracht sein und damit von der Tür 6 getragen werden. Vorzugsweise sind der Antennenumschalter 15 und die Leseelektronik 16 stattdessen jedoch nicht in der Tür sondern ebenfalls im Geräteschaltschrank des Spülautomaten 1, insbesondere in einer Steuereinheit angeordnet. Die Leseeinheit 10 ist dabei mit dem Antennenumschalter 15 und der Leseelektronik 16 mittels Kabel, insbesondere eines durch eine Schleppkette geschützten Koaxialkabels, verbunden. Hierdurch wird ein besonders robustes Detektionssystem realisiert.

Wie insbesondere die Darstellung nach Fig. 1 erkennen lässt, erfolgt mittels des erfindungsgemäßen Detektionssystems 8 ein Abtasten der im Spülraum 3 befindlichen Identifikatoren 9 durch die türseitig des Spülautomaten 1 angeordnete Leseeinheit 10. Nach einem ordnungsgemäßen Bestücken des Spülraums ist die Tür 6 verwenderseitig in Höhenrichtung 7 nach unten zu verfahren. Dank der Anordnung der Leseeinheit 10 an der Tür 6 wird so ein Vorbeibewegen der Leseeinheit 10 an der Beschickungsöffnung 5 und damit am Spülraum 3 sichergestellt. Infolge dieser Vorbeibewegung erfolgt mittels der Leseeinheit 10 ein Abtasten, das heißt Abscannen der einzelnen im Spülraum 3 befindlichen Identifikatoren 9, so dass eine fehlerfreie und vollständige Erfassung sämtlicher Identifikatoren 9 durch die Leseeinheit 10 erfolgt.

Die Leseeinheit 10 verfügt dabei über drei Planarantennen 13, die an einen gemeinsamen Antennenumschalter 15 angeschlossen sind. Dieser sorgt dank der Leseelektronik 16 dafür, dass die einzelnen Antennen 13 zeitversetzt angesteuert werden, womit ein vollständiges Abtasten des Spülraums 3 durch die Antennen 13 bei einer Verfahrbewegung der Tür 6 in Höhenrichtung 7 nach unten sichergestellt ist.

Die erfindungsgemäße Ausgestaltung erbringt u. a. folgende Vorteile:
- Automatische Auswahl des für das Behandlungsgut passenden Reinigungsprogramms (bzw. der Parameter)
- Ausbau der Prozessdokumentation
- Möglicher Aufbau von Controllingsystemen: Durchlaufzeiten, Bestandsreduzierung, Lagerkennzahlen, wie z. B. Umschlagshäufigkeit
- Steigerung der Prozesssicherheit; Qualitätssicherung durch lückenlose Prozessdokumentation, inkl. möglicher Verknüpfung mit einer Patientenakte
- Verknüpfung von Chargenprotokoll mit einzelnen Instrumenten sowie die möglicherweise anschließende Verknüpfung von angewandten Instrumenten mit der Patientenakte
- Ggf. Speicherung von Zusatzinfos auf Instrumenten (Durchlaufzähler, Wartungsintervalle, Aufbereitungshinweise)

Das System besteht nur aus sehr wenigen, robusten Einzelteilen, die auf einfache Art und Weise miteinander verbunden sind. Es gibt keine Verschleißteile. Der Wartungsbedarf ist äußerst gering. Gleichzeitig ist das System sehr unauffällig.

### Bezugszeichen

- 1: Spülautomat
- 2: Spülbehälter
- 3: Spülraum
- 4: Spülgut
- 5: Beschickungsöffnung
- 6: Tür
- 7: Höhenrichtung
- 8: Detektionssystem
- 9: Identifikator
- 10: Leseeinheit
- 11: Glasscheibe
- 12: Durchbruch
- 13: Antenne
- 14: Gehäuse
- 15: Antennenumschalter
- 16: Leseelektronik
- 17: Anschaltelektronik

## Patentansprüche

1. Spül-, Sterilisations- oder Waschautomat, insbesondere Reinigungs- und Desinfektionsgerät für medizinische Instrumente, mit einem einen Behandlungsraum (3) bereitstellenden Spül-, Sterilisations- oder Waschbehälter (2), der eine mittels einer Tür (6) fluiddicht verschließbare Beschickungsöffnung (5) aufweist, und mit einem Detektionssystem (8) zur automatischen Detektion von im Behandlungsraum (3) befindlichem Behandlungsgut (4),
wobei das Detektionssystem (8) einen im Behandlungsraum (3) anordbaren, berührungslos auslesbaren elektronischen Identifikator (9) sowie eine Leseeinheit (10) aufweist, wobei die Leseeinheit (10) relativ bewegbar zum Behandlungsraum (3) angeordnet ist,
**dadurch gekennzeichnet,**
**dass** die Leseeinheit (10) mehrere Antennen (13) aufweist, die derart im unteren oder einem seitlichen Bereich der Tür (6) angeordnet sind, dass sie beim Bewegen der Tür (6) von der vollständig geöffneten Stellung in die geschlossene Stellung am Behandlungsraum (3) nahezu über dessen gesamte Breite oder dessen gesamte Höhe entlangfahren können.

2. Spül-, Sterilisations- oder Waschautomat nach Anspruch 1, **dadurch gekennzeichnet, dass** der Identifikator (9) ein Transponder ist.

3. Spül-, Sterilisations- oder Waschautomat nach Anspruch 1 oder 2, **dadurch gekennzeichnet, dass** die Tür (6) einen mit einer Abdeckung, insbesondere einer Glasscheibe (11) fluiddicht verschlossenen Durchbruch (12) aufweist, wobei die Abdeckung, insbesondere die Glasscheibe (11) zwei unter Belassung eines Spaltraums beabstandet voneinander angeordnete Abdeckungselemente, insbesondere Einzelscheiben aufweist, wobei die Leseeinheit (10) im Spaltraum zwischen den Abdeckungselementen, insbesondere Einzelscheiben angeordnet ist.

4. Spül-, Sterilisations- oder Waschautomat nach einem der vorhergehenden Ansprüche, **dadurch gekennzeichnet, dass** die Leseeinheit (10) mehrere, vorzugsweise drei Planarantennen (13) aufweist.

5. Spül-, Sterilisations- oder Waschautomat nach einem der vorhergehenden Ansprüche, **dadurch gekennzeichnet, dass** die Leseeinheit (10) von einem Gehäuse (14) aufgenommen ist.

6. Spül-, Sterilisations- oder Waschautomat nach Anspruch 5, **dadurch gekennzeichnet, dass** das Gehäuse (14) auf seiner dem Behandlungsraum abgewandten Rückseite abgeschirmt ausgebildet ist.

7. Spül-, Sterilisations- oder Waschautomat nach Anspruch 6, **dadurch gekennzeichnet, dass** die Rückseite des Gehäuses (14) einen Durchbruch aufweist.

## Claims

1. Automatic rinsing, sterilising or washing machine, in particular a cleaning and disinfection device for medical instruments, comprising a rinsing, sterilising or washing container (2) that provides a treatment chamber (3) and comprises a loading opening (5) that can be closed in a fluid-tight manner by means of a door (6), and comprising a detection system (8) for automatically detecting when items (4) to be treated are in the treatment chamber (3),
the detection system (8) comprising an electronic identifier (9) that can be arranged in the treatment chamber (3) and read contactlessly, and a read unit (10), the read unit (10) being arranged so as to be movable relative to the treatment chamber (3),
**characterised in that**
the read unit (10) comprises a plurality of antennas (13) which are arranged in the lower or a side region of the door (6) such that they can move along the treatment chamber (3) almost over the entire width or the entire height thereof when the door (6) is moved from the completely open position into the closed position.

2. Automatic rinsing, sterilising or washing machine according to claim 1, **characterised in that** the identifier (9) is a transponder.

3. Automatic rinsing, sterilising or washing machine according to either claim 1 or claim 2, **characterised in that** the door (6) comprises a through-opening (12) that is closed in a fluid-tight manner by a cover, in particular a glass panel (11), the cover, in particular the glass panel (11), comprising two cover elements, in particular individual panels, which are arranged at a spacing from one another so as to leave a gap, the read unit (10) being arranged in the gap between the cover elements, in particular individual panels.

4. Automatic rinsing, sterilising or washing machine according to any of the preceding claims, **characterised in that** the read unit (10) comprises a plurality of planar antennas (13), preferably three planar antennas.

5. Automatic rinsing, sterilising or washing machine according to any of the preceding claims, **characterised in that** the read unit (10) is received in a housing (14).

6. Automatic rinsing, sterilising or washing machine according to claim 5, **characterised in that** the housing (14) is shielded on its rear side that faces away from the treatment chamber.

7. Automatic rinsing, sterilising or washing machine according to claim 6, **characterised in that** the rear side of the housing (14) comprises a through-opening.

## Revendications

1. Appareil automatique de rinçage, de stérilisation ou de lavage, en particulier appareil de nettoyage et de désinfection pour instruments médicaux, avec une cuve de rinçage, de stérilisation ou de lavage (2) qui fournit un espace de traitement (3) et qui présente une ouverture de chargement (5) pouvant être fermée de façon étanche aux fluides au moyen d'une porte (6), et avec un système de détection (8) destiné à la détection automatique d'articles à traiter (4) situés dans l'espace de traitement (3),
dans lequel le système de détection (8) présente un identificateur (9) électronique pouvant être disposé dans l'espace de traitement (3) et qui est lisible sans contact, ainsi qu'une unité de lecture (10), dans lequel l'unité de lecture (10) est disposée de façon mobile par rapport à l'espace de traitement (3),
**caractérisé en ce que**
l'unité de lecture (10) présente plusieurs antennes (13) qui sont disposées dans la zone inférieure ou dans une zone latérale de la porte (6) de telle sorte que, lors du mouvement de la porte (6) à partir de la position complètement ouverte jusqu'à la position fermée, elles peuvent longer l'espace de traitement (3) quasiment sur toute la largeur ou toute la hauteur de celui-ci.

2. Appareil automatique de rinçage, de stérilisation ou de lavage selon la revendication 1, **caractérisé en ce que** l'identificateur (9) est un transpondeur.

3. Appareil automatique de rinçage, de stérilisation ou de lavage selon la revendication 1 ou 2, **caractérisé en ce que** la porte (6) présente une traversée (12) fermée de façon étanche aux fluides par une couverture, en particulier par une vitre (11), dans lequel la couverture, en particulier la vitre (11), présente deux éléments de couverture, en particulier des vitres individuelles, disposées de façon espacée l'un de l'autre en laissant un espace interstitiel entre eux, dans lequel l'unité de lecture (10) est disposée dans l'espace interstitiel entre les éléments de couverture, en particulier les vitres individuelles.

4. Appareil automatique de rinçage, de stérilisation ou de lavage selon l'une des revendications précédentes, **caractérisé en ce que** l'unité de lecture (10) présente plusieurs, de préférence trois, antennes planes (13).

5. Appareil automatique de rinçage, de stérilisation ou de lavage selon l'une des revendications précédentes, **caractérisé en ce que** l'unité de lecture (10) est logée dans un boîtier (14).

6. Appareil automatique de rinçage, de stérilisation ou de lavage selon la revendication 5, **caractérisé en ce que** le boîtier (14) est constitué de façon blindée sur son côté arrière éloigné de l'espace de traitement.

7. Appareil automatique de rinçage, de stérilisation ou de lavage selon la revendication 6, **caractérisé en ce que** le côté arrière du boîtier (14) présente une traversée.
